# EUROPEAN PATENT SPECIFICATION

(11) **EP 0 458 279 B1**
(45) Date of publication and mention of the grant of the patent: **05.03.1997**
(21) Application number: 91108207.1
(22) Date of filing: 21.05.1991
(51) Int. Cl.: C11D 7/30

(54) **Cleaning solvent**
Lösungsmittel zum Reinigen
Solvant pour nettoyage

(30) Priority: 21.05.1990 JP 130453/90
(43) Date of publication of application: 27.11.1991
(73) Proprietor: Japan as represented by Director-General, Agency of Industrial Science and Technology, Tokyo 100 (JP); NIPPON ZEON CO., LTD., Chiyoda-ku Tokyo 100 (JP)
(72) Inventor: Sekiya, Akira, Tsukuba-shi, Ibaragi-ken (JP); Tamura, Masanori, Tsukuba-shi, Ibaragi-ken (JP); Shibakami, Motonari, Tsukuba-shi, Ibaragi-ken (JP); Yamada, Toshiro, Fujisawa-shi, Kanagawa-ken (JP); Goto, Kuniaki, Setagaya-ku, Tokyo (JP)
(74) Representative: VOSSIUS & PARTNER

(56) References cited:
- EP-A- 0 315 783
- US-A- 2 456 028

## Description

This invention relates to the use of a fluorinated cyclopentane or a mixture of fluorinated cyclopentanes as a cleaning solvent. The cleaning solvent has good dissolving power and cleaning action, which includes no risk of depletion of the ozone layer because it does not contain a chlorine atom and which can be used in the mixed state.

Halogenated hydrocarbons have been heretofore used as cleaning solvents for plastic and rubber articles and electronic parts, and they have characteristics as hydrocarbons containing chlorine atoms, or chlorine and fluorine atoms. These hydrocarbons are incombustible, low-toxic and stable, and they can dissolve waxes, fats and oils therein without corroding surfaces of plastic and rubber articles. Accordingly, they are widely used in various industrial fields.

As the hydrocarbons of this type, there are known chlorine-containing compounds such as trichloroethylene and tetrachloroethylene, and Freon® type compounds such as trifluorotrichloroethane (Freon® 113). The latter Freon® type hydrocarbons are especially low-toxic, and therefore, they have a very broad range of application.

It is apprehended, however, that the halogenated hydrocarbons having these characteristics, especially the chlorine-containing Freon® type compounds applicable in a broad range, will cause an environmental problem of depletion of the ozone layer surrounding the earth, and the trend of international opinion is to reduce the quantities used of these compounds or inhibit the use of these compounds.

To cope with this problem, various alternatives for Freon® 113 have been practically investigated, but no effective substance has been found. As the means for reducing the amount used of Freon® type compounds, mixed use of these compounds with other organic solvent has been proposed and practically used. Nevertheless, this mixed use has a problem in that the cleaning action is not strong and the application embodiment including recovery and repeated use is not practical.

A primary object of the present invention is to use a cleaning solvent which can solve the problem of a conventional cleaning solvent composed of a Freon® type compound which is low-toxic and easy to handle, that is, depletion of the ozone layer because of the presence of chlorine atoms. The used cleaning solvent is incombustible, has good dissolving power and cleaning action, retains a merit of a low toxicity and does not include any risk of depletion of the ozone layer because of the absence of chlorine atoms.

This object has been achieved by the use of
a cleaning solvent comprising a fluorinated cyclopentane represented by the following formula (I):

C₅H₁₀₋ₙFₙ (I)

wherein n is an integer of from 5 to 8.

Particularly the above-mentioned fluorinated cyclopentane is used as a cleaning solvent for cleaning a plastic or rubber article or an electronic part by placing the plastic or rubber article or the electronic part in contact with the above-mentioned fluorinated cyclopentane.

The fluorinated cyclopentane used as the cleaning solvent in the present invention has a structure formed by substituting hydrogen atoms by fluorine atoms in cyclopentane used as a diluent or the like in various fields, and the fluorinated cyclopentane has a good action as a cleaning solvent for plastic and rubber articles and electronic parts.

The fluorinated cyclopentane used in the present invention is characterized in that this can be used not only in the form of a single compound but also in the form of a mixture of compounds of the formula C₅H₁₀₋ₙFₙ, F , which have different n values in the range from 5 to 8.

The fluorinated cyclopentane used in the present invention can be used as the sole cleaning solvent for cleaning plastic articles, rubber articles and electronic parts, for example, plastics for printed circuit boards, but if desired, the fluorinated cyclopentane can be used in the form of mixtures with various solvents customarily used for Freon® 113, whereby their dissolving power can be increased and fluxes, oils and fats can be effectively removed. Alcohols can be mentioned as typical examples of such solvents. The cleaning of plastic articles, rubber articles and electronic parts with the fluorinated cyclopentane of the present invention is carried out usually at a temperature of 0 to 100°C for 1 second to 5 hours.

When the fluorinated cyclopentane is used as the cleaning solvent, various stabilizers can be incorporated in the fluorinated cyclopentane. For example, there can be mentioned aliphatic nitro compounds such as nitromethane and nitroethane, acetylene alcohols such as 3-methyl-1-butyn-3-ol and 3-methyl-1-pentyn-3-ol, epoxides such as glycidol, methyl glycidyl ether and allyl glycidyl ether, ethers such as dimethoxymethane and 1,4-dioxane, unsaturated hydrocarbons such as hexene, heptene, cyclopentene and cyclohexene, unsaturated alcohols such as allyl alcohol and 1-buten-3-ol, and acrylic acid esters such as methyl acrylate and ethyl acrylate.

Furthermore, to obtain a synergistic stabilizing effect, phenols, amines and benzotriazoles can be used in combination with the stabilizers.

A diluent can be used together with a stabilizer. For example, saturated hydrocarbons such as cyclopentane, cyclohexane, heptane and hexane can be mentioned as the diluent.

The process for the preparation of the fluorinated cyclopentane has already been known. For example, a process is known which comprises reacting cyclopentane as the starting material with a metal fluoride having a higher atomic valency such as cobalt trifluoride. According to this process, the hydrogen atoms of cyclopentane are substituted with fluorine atoms and a mixture of various fluorinated cyclopentanes is obtained. In the mixture, the average number of fluorine atoms in the fluorinated cyclopentanes is usually in the range from 6 to 8. This mixture can be used directly as the cleaning solvent of the present invention. A mixture having a desired boiling point or a compound having a desired substitution number of fluorine atoms can be obtained by controlling the reaction conditions or subjecting the reaction product to fractional distillation.

The fluorinated cyclopentane used in the present invention has a good cleaning action as the solvent. The fluorinated cyclopentane is thermally stable when used, and the stability is comparable to that of the conventional Freon® type compounds and the cleaning solvent of the present invention does not give any bad influence such as swelling and corrosion to plastic and rubber articles.

The fluorinated cyclopentane used in the present invention is incombustible and moreover, it is decomposable because of the presence of bound hydrogen atoms. Still further, since the fluorinated cyclopentane does not contain a chlorine atom, there is no risk of depletion of the ozone layer as a global environmental problem.

The present invention will now be described in detail by the following examples of the preparation and evaluation of the fluorinated cyclopentane that by no means limit the present invention.

### Referential Examples

### Preparation of fluorinated cvclopentane

(1) A stainless steel reactor having an inner capacity of 1 liter was charged with 2 moles of cobalt trifluoride and 60 millimoles of cyclopentane, and reaction was carried out at 200°C for 2 hours. Hydrogen fluoride produced as a by-product was removed, and then, the reaction product was subjected to simple distillation to obtain 10 g of a product. When the product was analyzed by the gas chromatography, it was found that the average introduction number of fluorine atoms into cyclopentane was 6.7.
The average introduction number of fluorine atoms is a weighted mean value of numbers of fluorine atoms in one molecule of respective constituents of the mixture according to the molar proportions of the respective constituents.
(2) Then 90 g of a reaction product obtained according to the above-mentioned process was subjected to distillation under atmospheric pressure by using a distillation column having a theoretical stage number of 5 to separate the reaction product into three fractions. The weights and boiling points of the respective fractions were as shown in Table 1, and the average introduction numbers of fluorine atoms and the compositions were as shown in Table 2.

**Table 1**

| Fraction No. | Weight(g) | Boiling point(°C) |
|---|---|---|
| 1 | 26.9 | 30 - 47 |
| 2 | 22.5 | 47 - 53 |
| 3 | 23.2 | 53 - 68 |

**Table 2**

| Charged fraction | Composition (mole%)*2 | | | | | | Av. No. of F atoms*1 |
|---|---|---|---|---|---|---|---|
| | F5 | F6 | F7 | F8 | F9 | F10 | |
| Charged amount | 10.0 | 29.6 | 38.9 | 18.9 | 2.3 | 0.3 | 6.7 |
| Fraction No. 1 | 1.0 | 10.7 | 34.5 | 46.4 | 6.7 | 0.7 | 7.5 |
| Fraction No. 2 | 5.1 | 29.8 | 53.0 | 12.0 | 0.1 | 0 | 6.7 |
| Fraction No. 3 | 16.4 | 43.5 | 37.4 | 2.7 | 0 | 0 | 6.2 |

| | | | | | | | |
|---|---|---|---|---|---|---|---|
| Note: *1 Average number of fluorine atoms in the fluorinated cyclopentanes. | | | | | | | |
| *2 F₅ through F₁₀ indicate fluorinated cyclopentanes having the indicated number of fluorine atoms in the molecule. For example, F₅ and F₆ indicate C₅F₅H₅ and C₅F₆H₄, respectively. | | | | | | | |

### Example 1

The test of cleaning a flux for a printed circuit board was carried out by using the fractions obtained in the referential example according to the following procedure.

A flux (H-10F supplied by San-Hayato Kabushiki Kaisha) was coated on a smooth glass sheet and dried at 30°C for 30 minutes. Then the coated glass sheet was immersed in the fraction for 30 seconds and the flux-removing effect was observed with the naked eye. In the case of fraction No. 3, the flux was removed as completely as in the case of Freon® 113. In the case of fractions No. 1 and No. 2, a very minute amount of the flux was left, but the cleaning effect was satisfactory.

### Example 2

Influences of the respective fractions obtained in the referential example on a substrate to be coated thereon were tested according to the following procedures.

In a sealed vessel, a polypropylene test piece (5 mm x 50 mm x 2 mm) was immersed in the fraction and the test piece was allowed to stand at 50°C in this state for 4 hours. Then the weight change was determined. In all of the fractions, the weight change was smaller than 1%.

For reference, the test was similarly carried out by using Freon® 113. It was found that the weight change was smaller than 1%.

### Example 3

The thermal stability test was carried out with respect to each of the fractions obtained in the referential example. It was found that in each fraction, the decomposition-initiating temperature was in the range of from about 300°C to about 400°C, almost comparable to the level of Freon® 113.

## Claims

1. Use of a fluorinated cyclopentane or a mixture of fluorinated cyclopentanes represented by the following formula (I):
C₅H₁₀₋ₙFₙ (I)
wherein n is an integer from 5 to 8 as a cleaning solvent

2. Use according to claim 1, wherein the average number of fluorine atoms in the fluorinated cyclopentanes in said mixture is from 6 to 8.

3. Use according to claim 1 or 2 for cleaning an electronic part, a plastic article or a rubber article.

## Patentansprüche

1. Verwendung eines fluorierten Cyclopentans oder eines Gemisches fluorierter Cyclopentane der folgenden Formel (I):
C₅H₁₀₋ₙFₙ (I)
in der n eine ganze Zahl von 5 bis 8 ist, als Reinigungslösungsmittel.

2. Verwendung nach Anspruch 1, wobei die durchschnittliche Zahl der Fluoratome in den fluorierten Cyclopentanen im Gemisch 6 bis 8 beträgt.

3. Verwendung nach Anspruch 1 oder 2 zur Reinigung eines elektronischen Teils, eines Kumtoffartikels oder eines Kautschukanikels.

## Revendications

1. Utilisation d'un cyclopentane fluoré ou d'un mélange de cyclopentanes fluorés représentés par la formule (I) suivante :
C₅H₁₀₋ₙFₙ (I)
dans laquelle n est un nombre entier de 5 à 8 comme agent de nettoyage.

2. Utilisation selon la revendication 1, dans laquelle le nombre moyen d'atomes de fluor dans les cyclopentanes fluorés dans ledit mélange est de 6 à 8.

3. Utilisation selon la revendication 1 ou 2 pour nettoyer une partie électronique, un article en plastique ou un article en caoutchouc.
